# EUROPEAN PATENT SPECIFICATION

(11) **EP 1 808 165 B1**
(45) Date of publication and mention of the grant of the patent: **29.04.2009**
(21) Application number: 06251325.4
(22) Date of filing: 14.03.2006
(51) Int. Cl.: A61K 9/20, A61K 31/496

(54) **Dry formulations of aripiprazole**
Trockene Aripiprazolformulierungen
Formulations sèches d'aripiprazole

(30) Priority: 05.01.2006 US 756707 P
(43) Date of publication of application: 18.07.2007
(73) Proprietor: Teva Pharmaceutical Industries Ltd, 49131 Petah Tiqva (IL)
(72) Inventor: Hrakovsky, Julia, Rosh Ha-Ayin 48057 (IL); Tenengauzer, Ruth, Hod Hasharon 45322 (IL)
(74) Representative: Nachshen, Neil Jacob

(56) References cited:
- EP-A- 1 475 084
- WO-A-03/026659
- WO-A-20/05058835
- US-A1- 2005 019 398
- US-A1- 2005 202 088

## Description

### Field of the Invention

The invention encompasses methods of making dry compression formulations of anhydrous aripiprazole Type-I, Type-II or Form-II, and dry formulation tablets made by such methods.

### Background of the invention

Aripiprazole, as reported in the literature, can exist in multiple crystal forms. For example, PCT publication WO 03/026659 describes at least nine crystal forms, including an hydrate and anhydrous forms, such as Type-I and Type-II. According to WO 03/026659, the procedures disclosed in Proceedings of the 4th Japanese-Korean Symposium on Separation Technology (October 6-8, 1996) yield significantly hydroscopic crystalline forms. The procedures disclosed in the Proceedings yield Type-I crystals of aripiprazole anhydride, prepared by recrystallizing from an ethanol solution of aripiprazole, or by heating aripiprazole hydrate at 80°C. The same Proceedings disclose that Type-II crystals of aripiprazole anhydride can be prepared by heating Type-I crystals of aripiprazole anhydride at 130°C to 140°C for 15 hours. In addition to Type-I and Type-II crystals, several additional anhydrous crystal forms are known. PCT publication WO 03/026659 discloses anhydride crystals Form B, C, D, E, F, or G and a hydrate form denominated Form A.

As reported in WO 03/026659, the multiple polymorphs may interconvert from one to the other. For instance, WO 03/026659 discloses that if the anhydrous form is exposed to moisture, then it may take on water and convert into a hydrous form. As stated in WO 03/026659, this presents several disadvantages, for instance the compound may be less bioavailable and less soluble. The hygroscopicity of aripiprazole crystals makes them difficult to handle since costly and burdensome measures must be taken to ensure that the crystals are not exposed to moisture during process and formulation. Despite these concerns, WO 03/026659 discloses a wet granulation process for preparing pharmaceutical compositions using aripiprazole anhydride and various carriers.

Other novel crystal aripiprazole forms are disclosed in PCT publication WO 05/058835. These other forms include Form I, II, VII, VIII, X, XI, XII, XIV, XIX, and XX.

Polymorphic transformations may be undesirable during pharmaceutical composition preparation or formulation. Hydration or manipulation of polymorphs may induce such unwanted polymorphic transformations. Also, the use of some aripiprazole polymorphs in pharmaceutical tablets may potentially induce unwanted polymorphic transformations, which in turn may reduce the bioavailability of the drug. Therefore, it would be desirable to develop aripiprazole formulations in which there is no potential of hydration and/or possible polymorphic interconversions.

### Summary of the invention

One embodiment of the invention encompasses a method of making a formulation of at least one of anhydrous aripiprazole Type-I, Type-II or Form II comprising providing a mixture of aripiprazole, at least one diluent, at least one tablet binder, and at least one tablet disintegrant; blending the mixture to obtain a homogeneous mixture; optionally adding at least one tablet lubricant to the homogeneous mixture; and dry compressing the homogeneous mixture into the formulation. The formulation may be tablets, slugs or a compact. The method may further comprise milling the slug or compact into a granulate, adding at least one tablet lubricant to the granulate, and dry compressing the granulate into a tablet. The mixture may further comprise a colorant.

The aripiprazole is at least one of anhydrous aripiprazole Type-I, Type-II, or Form II. In one particular embodiment, the aripiprazole may have a particle size distribution where d(0.9) is about 300 µm or less. The tablet may have a dissolution rate where not less than 80% of the initial aripiprazole is dissolved after about 30 minutes. Preferably, the tablet may have a dissolution rate where not less than 85% of the initial aripiprazole is dissolved after about 30 minutes, and more preferably not less than 90%, as tested under the conditions described below.

In another embodiment, the diluent is calcium carbonate, calcium phosphate (dibasic and/or tribasic), calcium sulfate, powdered cellulose, dextrates, dextrin, fructose, kaolin, lactitol, anhydrous lactose, lactose monohydrate, maltose, mannitol, microcrystalline cellulose, sorbitol, sucrose, or starch. Preferably, the diluent is lactose monohydrate, microcrystalline cellulose, or starch. In one particular embodiment, the diluent is present in an amount of about 35% to about 85% by weight of the tablet.

In another embodiment, the binder is acacia, alginic acid, carbomer, sodium carboxymethylcellulose, dextrin, ethylcellulose, gelatin, glucose, guar gum, hydroxypropyl cellulose, maltose, methylcellulose, polyethylene oxide, or povidone. Preferably, the binder is hydroxypropyl cellulose. In one particular embodiment, the binder is present in an amount of about 0.5% to about 5% by weight of the tablet.

In yet another embodiment, the disintegrant is alginic acid, sodium croscarmellose, crospovidone, maltose, microcrystalline cellulose, potassium polacrilin, sodium starch glycolate, or starch. Preferably, the disintegrant is crospovidone, sodium starch glycolate or sodium croscarmellose. In one particular embodiment, the disintegrant is present in an amount of about 3% to about 15% by weight of the tablet.

In yet another embodiment, the lubricant is calcium stearate, glyceryl behenate, magnesium stearate, mineral oil, polyethylene glycol, sodium stearyl fumarate, stearic acid, talc, or zinc stearate. Preferably, the lubricant is magnesium stearate. In one particular embodiment, the lubricant is present in an amount of about 0.5% to about 2% by weight of the tablet.

In one embodiment, the tablet comprises: aripiprazole Type-I, lactose monohydrate, starch, microcrystalline cellulose, hydroxypropyl cellulose, and magnesium stearate.

In another embodiment, the tablet comprises aripiprazole Type-II, lactose monohydrate, starch, microcrystalline cellulose, hydroxypropyl cellulose, color red, and magnesium stearate.

In yet another embodiment, the tablet comprises aripiprazole Form II, lactose monohydrate, starch, microcrystalline cellulose, hydroxypropyl cellulose, sodium starch glycolate, color red, and magnesium stearate.

### Brief Description of the Figures

Figure 1 illustrates the x-ray diffraction pattern of aripiprazole Type-I.
Figure 2 illustrates the x-ray diffraction pattern of aripiprazole Type-II.

### Detailed Description of the Invention

The problems associated with the hydration of aripiprazole during formulation or storage have focused research into developing stable anhydrous forms of aripiprazole. These forms would be less or non-hygroscopic, and thus resistant to hydration and the accompanying possible polymorphic transformation. The present invention provides an alternative to the development of stable anhydrous forms of aripiprazole. The present invention encompasses methods of making tablets using dry formulations in direct compression or dry granulation via dry compaction. These dry formulations and the methodology associated with such dry formulations prevent or reduce hydration and the associated subsequent polymorphic transformations.

Thus, the present invention encompasses methods of making tablets by compression of dry formulations and tablets made using dry compression methodology. There are economic advantages in the dry compression of formulations over wet granulation, because the dry compression requires fewer unit operations. Using less equipment, lower power consumption, less space, less time, and less labor are a few examples of how the methodology reduces production cost of tablets. Also, dry compression avoids the use of organic solvents during the preparation of the formulations. Organic solvents may be either toxic or difficult to dispose of because of environmental concerns.

Dry compression, however, is generally limited to those circumstances in which the active ingredient has physical characteristics suitable for forming pharmaceutically acceptable tablets. These physical characteristics include, but are not limited to, good flowing properties, compressibility, and compactability.

Dry compression formulations comprising aripiprazole were developed, because it was found that aripiprazole crystals were suitable for dry compression formulations. In particular, it was found that anhydrous aripiprazole crystals were suitable for dry compression formulations. As used herein with the term "aripiprazole," the term "anhydrous" means aripiprazole is crystallized in a form, which does not contain solvent of crystallization or water incorporated within the crystal lattice, but may include water outside the crystal lattice.

The method of making an aripiprazole formulation comprises providing a mixture of aripiprazole, at least one diluent, at least one tablet binder, and at least one tablet disintegrant; blending the mixture to obtain a homogeneous mixture; optionally adding at least one tablet lubricant to the homogeneous mixture; and dry compressing the homogeneous mixture into the formulation. The formulation can be in the shape of a tablet, a slug, or a compact. The method may further comprise milling the slug or compact into a granulate, adding at least one tablet lubricant to the milled granulate, and dry compressing the milled granulate into a tablet. Optionally, at least one colorant may be added to the mixture to provide any desired colored tablet.

The blending step is carried out to substantially homogeneous mixture. The skilled artisan with little or no experimentation can easily determine the equipment and conditions necessary for the blending steps. Factors that may influence the blending step include, but are not limited to, the amount of materials, the physical characteristics of the materials, the equipment, and the speed of mixing.

The dry compressing step includes compressing the homogeneous mixture into a formulation. The formulation may be shaped as tablets, ribbons or blocks of solid material, or slugs. When the formulation is shaped as ribbons or blocks of solid material, or slugs, the ribbons or blocks of solid material, or slugs are milled. Thereafter, the milled material or granulate is blended with extragranular excipients and compressed into tablets. The compressing step may be carried out using a tablet compression apparatus commonly used in tableting or other suitable equipment to make slugs, ribbons, or blocks of solid material. For example, a Kilian tableting press may be used to form the tablets.

Anhydrous aripiprazole is used in the dry compression or dry granulation formulation. The anhydrous aripiprazole is at least one of Type-I, Type-II, or Form II. Type-I aripiprazole may be prepared by crystallization in ethanol and drying according to method described in WO 2005/058835. Alternatively, Type-I aripiprazole may be made according to the Reference Examples of WO 03/026659 and as described in the Proceedings of the 4^{th} Japanese-Korean Symposium on Separation Technology (October 6-8, 1996). Type-II may be obtained by heating Type-I crystals of aripiprazole anhydride at 140°C for 15 hours, according to the Reference Examples disclosed WO 03/026659. Form II aripiprazole may be prepared as disclosed in WO 05/058835.

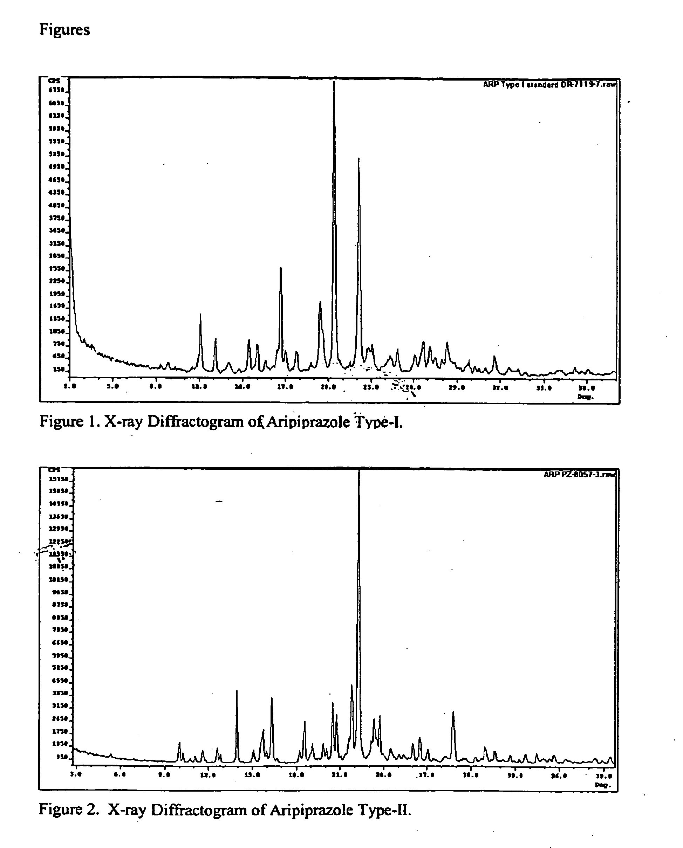

Type-I aripiprazole is characterized by x-ray diffraction peaks at 8.8, 10.6, 11.1, 12.1, 15.0, 15.8, 17.7, 20.4, 22.1, and 29.8 ± 0.2 degrees 2-theta. Type-II aripiprazole is characterized by x-ray diffraction peaks at 10.1, 11.7, 13.9, 15.1, 18.2, 20.8, 21.8, 23.5, 23.8, and 28.9 ± 0.2 degrees 2-theta. The XRD diffractograms of aripiprazole Type-I and Type-II are shown in figures 1 and 2, respectively. Form II aripiprazole is characterized by x-ray diffraction peaks at 16.5, 18.7, 21.9, 22.4, and 23.5 ± 0.2 degrees 2-theta.

The crystal form of aripiprazole within the pharmaceutical compositions may be monitored using known state of the art techniques. For example, techniques such as X-ray powder diffraction (XRD) or solid-state NMR of carbon-13, nitrogen-14, or chlorine, among others, may be used. Generally, any instrumentation of X-Ray powder diffraction or solid-state NMR normally available in laboratories is suitable for monitoring the crystal forms of aripiprazole in pharmaceutical compositions. Typical methods for obtaining X-ray diffractions of aripiprazole may be found in WO 03/026659 or WO 05/058835.

Optionally, the aripiprazole may have a particle shape. Typically, the particle size distribution d(0.9) is about 300 µm or less. If aripiprazole Type-I or Type-II is used, the particle size distribution d(0.9) is about 180 µm to about 270 µm. If aripiprazole Form II is used, the particle size distribution d(0.9) is about 25 µm.

The single dose of the active ingredient is small, and an inert substance may be added to increase the bulk and make the tablet a practical size for compression. Diluents are used for this purpose. Diluents used in the mixture include diluents commonly used for tablet preparation. For example, diluents include, but are not limited to, calcium carbonate, calcium phosphate (dibasic and/or tribasic), calcium sulfate, powdered cellulose, dextrates, dextrin, fructose, kaolin, lactitol, anhydrous lactose, lactose monohydrate, maltose, mannitol, microcrystalline cellulose, sorbitol, sucrose, or starch. Preferably, the diluent is lactose monohydrate, microcrystalline cellulose, or starch. Typically, the diluent is present in an amount of about 35% to about 85% by weight of the tablet. Preferably, the diluent is present in an amount of about 40% to about 80% by weight of the tablet.

Binders are agents used to impart cohesive qualities to the powdered material. Binders impart a cohesiveness to the tablet formulation that ensures that the tablet remains intact after compression. Tablet binders used in the mixture include tablet binders commonly used for tablet preparation. Tablet binders include, but are not limited to, acacia, alginic acid, carbomer, sodium carboxymethylcellulose, dextrin, ethylcellulose, gelatin, glucose, guar gum, hydroxypropyl cellulose, maltose, methylcellulose, polyethylene oxide, or povidone. Preferably, the tablet binder is hydroxypropyl cellulose. Typically, the tablet binder is present in an amount of about 0.5% to about 5% by weight of the tablet. Preferably, the tablet binder is present in an amount of about 0.7% to about 3% by weight of the tablet.

A disintegrant is a substance or mixture of substances added to a tablet formulation to facilitate a tablet's breakup or disintegration after tablet administration. The aripiprazole should be released from the tablet as efficiently as possible to allow dissolution. Tablet disintegrants used in the mixture include, but are not limited to, alginic acid, sodium croscarmellose, crospovidone, maltose, microcrystalline cellulose, potassium polacrilin, sodium starch glycolate, or starch. Preferably, the tablet disintegrant is a "super-disintegrant:" crospovidone, sodium starch glycolate or sodium croscarmellose. Typically, the tablet disintegrant is present in an amount of about 3% to about 15% by weight of the tablet. Preferably, the tablet disintegrant is present in an amount of about 5% to about 10% by weight of the tablet.

Lubricants have a number of functions in tablet manufacturing. For example, lubricants prevent adhesion of the tablet material to equipment, reduce interparticle friction, and facilitate the ejection of the tablet from the die cavity, among others. Tablet lubricants added to the homogeneous mixture include those typically used in tablet formulations. Tablet lubricants include, but are not limited to, calcium stearate, glyceryl behenate, magnesium stearate, mineral oil, polyethylene glycol, sodium stearyl fumarate, stearic acid, talc, or zinc stearate. Preferably, the tablet lubricant is magnesium stearate. Typically, the tablet lubricant is present in an amount of about 0.5 to about 2 percent by weight of the tablet. Preferably, the tablet lubricant is present in an amount of about 0.7 to about 1 percent by weight of the tablet.

In one embodiment, the dry compression pharmaceutical formulation made by a method of the invention has a dissolution rate where not less than 80% of the initial aripiprazole is dissolved after about 30 minutes. Preferably, the tablet may have a dissolution rate where not less than 85% of the initial aripiprazole is dissolved after about 30 minutes, and more preferably not less than 90%.

Once a tablet was made using the methodology described above, the aripiprazole was tested to determine whether a polymorphic transformation had occurred. The x-ray diffraction pattern of the aripiprazole within the pharmaceutical composition made in Example 1 had peaks at 8.8, 10.6, 11.1, 12.1, 15.0, 15.8, 17.7, 22.1, and 29.8 ± 0.2 degrees 2-theta. The x-ray diffraction pattern of the aripiprazole within the pharmaceutical composition made in Example 2 had peaks at 10.1, 11.7, 14.0, 15.1, and 21.9 ± 0.2 degrees 2-theta. A comparison of the x-ray diffraction patterns of the aripiprazole of Examples 1 and 2 with the x-ray diffraction patterns with aripiprazole Type-I and Type-II, respectively, demonstrated that the tablet obtained by dry compression of the dry aripiprazole formulation did not include other polymorphic aripiprazole forms, including hydrates.

The invention also encompasses tablets made using the methodology described above. In one embodiment the tablet comprises aripiprazole Type I, Type II or Form II, lactose monohydrate, starch, microcrystalline cellulose, hydroxypropyl cellulose, and magnesium stearate. Optionally, the tablet may further comprise a colorant. In another embodiment the tablet comprises aripiprazole Type-I, lactose monohydrate, starch, microcrystalline cellulose, hydroxypropyl cellulose, and magnesium stearate. In a preferred embodiment the tablet comprises aripiprazole Type-I (30 mg/tablet), lactose monohydrate (120 mg/tablet), starch (60 mg/tablet), microcrystalline cellulose (60 mg/tablet), hydroxypropyl cellulose (8 mg/tablet), and magnesium stearate (2 mg/tablet).

In yet another embodiment the tablet comprises aripiprazole Type-II, lactose monohydrate, starch, microcrystalline cellulose, hydroxypropyl cellulose, color red, and magnesium stearate. In a preferred embodiment, the tablet comprises aripiprazole Type-II (30 mg/tablet), lactose monohydrate (120 mg/tablet), starch (60 mg/tablet), microcrystalline cellulose (60 mg/tablet), hydroxypropyl cellulose (8 mg/tablet), color red (0.06 mg/tablet), and magnesium stearate (2 mg/tablet).

Another embodiment the tablet comprises aripiprazole Form II, lactose monohydrate, starch, microcrystalline cellulose, hydroxypropyl cellulose, sodium starch glycolate, color red, and magnesium stearate. Preferably, the tablet comprises aripiprazole Form II (30 mg/tablet), lactose monohydrate (112 mg/tablet), starch (60 mg/tablet), microcrystalline cellulose (94 mg/tablet), hydroxypropyl cellulose (2 mg/tablet), sodium starch glycolate (10 mg/tablet), color red (0.06 mg/tablet), and magnesium stearate (2 mg/tablet).

In a preferred embodiment, the method comprises blending aripiprazole Type-I, lactose monohydrate, starch, color red, hydroxypropyl cellulose, and magnesium stearate into a mixture; dry granulating the mixture and compressing the granulated mixture into slugs; milling the slugs and blending the milled slugs with microcrystalline cellulose and magnesium stearate into a second mixture; compressing the second mixture into tablets, wherein the tablets have a hardness range of about 9 to 15 Strong-Cobb units and a friability of less than about 1%. Optionally, the aripiprazole Type-I has a d(0.9) value of about 186 µm.

It is understood, of course, that some excipient materials can function as both diluent and binder, or filler and disintegrant, and that some materials may exist that can fulfill all three roles. There is no intention to limit the invention to methods only using three distinct excipient materials "diluent," "tablet binder," and "tablet disintegrant," but rather the invention is directed to materials fulfilling these functions. For example the material that is the "at least one diluent" also might be the same as the material fulfilling the role of "at least one tablet binder" as long as the material is present in sufficient amount to fulfill both functions.

Another embodiment the tablet comprises aripiprazole Type I, lactose monohydrate, starch, microcrystalline cellulose, hydroxypropyl cellulose, color red, and magnesium stearate. Preferably, the tablet comprises aripiprazole Type I (30 mg/tablet), lactose monohydrate (106.44 mg/tablet), starch (60 mg/tablet), microcrystalline cellulose (81 mg/tablet), hydroxypropyl cellulose (3 mg/tablet), color red (0.06 mg/tablet), and magnesium stearate (4.5 mg/tablet).

Having described the invention with reference to certain preferred embodiments, other embodiments will become apparent to one skilled in the art from consideration of the specification. The invention is further defined by reference to the following examples describing in detail the formation of dry compression pharmaceutical formulations of aripiprazole and the dissolution of the tablets made using the dry compression pharmaceutical formulations. It will be apparent to those skilled in the art that many modifications, both to materials and methods, may be practiced without departing from the scope of the invention.

### Examples

### Example 1: Preparation of 30 mg Tablets Containing Aripiprazole Type-I Using Dry Compression

A mixture was made of aripiprazole Type-I (105 g), lactose monohydrate NF (420 g), starch NF (210 g), microcrystalline cellulose NF (210 g), and hydroxypropyl cellulose NF (28 g). The aripiprazole Type-I had a D(0,9) value of about 245 µm. The mixture was blended for 20 minutes. Magnesium stearate NF (7 g) was sieved and added to the blended mixture and blended for an additional 5 minutes. Thereafter, the mixture was compressed into tablets using a Kilian tableting press to have a hardness range of about 12 to 22 Strong-Cobb units and a friability of less than 1%.

### Example 2. Preparation of 30 mg Tablets Containing Aripiprazole Type-II Using Dry Compression

A mixture was made of aripiprazole Type-II (120 mg), lactose monohydrate NF (479.76 g), starch NF (240 g), microcrystalline cellulose NF (240 g), hydroxypropyl cellulose NF (32 g), and color red (0.24 g). The aripiprazole Type-II had a D(0,9) value of about 270 µm. The mixture was blended for 20 minutes. Magnesium stearate NF (8 g) was sieved and added to the blended mixture and blended for an additional 5 minutes. Thereafter, the mixture was compressed into tablets using a Kilian tableting press to have a hardness range of about 8 to 21 Strong-Cobb units and a friability of less than 1%.

### Example 3. Preparation of 30 mg Tablets Containing Aripiprazole Form II Using Dry Compression

A mixture was made of aripiprazole Form II (150 g), lactose monohydrate NF (559.7 g), starch NF (150 g), microcrystalline cellulose NF (470 g), hydroxypropyl cellulose NF (10 g), sodium starch glycolate (50 g), and color red (0.3 g). The mixture was blended for 20 minutes. Magnesium stearate NF (10 g) was sieved and added to the blended mixture and blended for an additional 5 minutes. Thereafter, the mixture was compressed into tablets using a Kilian tableting press to have a hardness range of about 5 to 25 Strong-Cobb units and a friability of less than 1%.

### Example 4. Preparation of 30 mg Tablets Containing Aripiprazole Type I Using a Dry Granulation Method

A mixture of aripiprazole Type-I (210 g), lactose monohydrate NF (745.08 g), starch NF (420 g), color red (0.42 g), hydroxypropyl cellulose NF (21 g) and magnesium stearate NF (15.75 g) was dry granulated. The aripiprazole Type-I had a D(0,9) value of about 186 µm. The mixture was compressed into slugs, the slugs were milled and blended with extragranular excipients: microcrystalline cellulose NF (567 g) and magnesium stearate NF (15.75 g). Thereafter, the mixture was compressed into tablets using a Kilian tableting press to have a hardness range of about 9 to 15 Strong-Cobb units and a friability of less than 1%.

### Example 5: Dissolution Measurements of Tablets Made in Examples 1-4

The dissolution for tablets from each of the above-described examples was studied. Typically, a the dissolution rate was measured for each batch after 30 minutes. The dissolution was carried out using an USP apparatus II (paddle) at 60 rpm with 900 ml of 0.1 N HCl at a temperature of 37°C. The results are summarized in Table 1.

| Table 1, Dissolution Measurement of Tablets from Examples 1-4 | | | |
|---|---|---|---|
| Example No. | Time (minutes) | Average Dissolution* | Minimum Dissolution* |
| 1 | 30 | 95 | 90 |
| 2 | 30 | 85 | 80 |
| 3 | 30 | 87 | 81 |
| 4 | 30 | 98 | 96 |

| | | | |
|---|---|---|---|
| * Average dissolution and minimum dissolution are reported as a percent by weight of the labeled amount. | | | |

## Claims

1. A method of making a formulation of at least one of anhydrous aripiprazole Type-I, Type-II, or Form II comprising:
providing a mixture of aripiprazole, at least one diluent, at least one tablet binder, and at least one tablet disintegrant;
blending the mixture to obtain a homogeneous mixture;
optionally adding at least one tablet lubricant to the homogeneous mixture; and
dry compressing the homogeneous mixture into the formulation.

2. The method according to claim 1, wherein the formulation is a tablet.

3. The method according to claim 1, the formulation is a slug or a compact.

4. The method according to claim 3, further comprising milling the slug or compact into a granulate, adding at least one tablet lubricant to the granulate, and dry compressing the granulate into a tablet.

5. The method according to any one of the preceding claims, wherein the mixture further comprises a colorant.

6. The method according to any one of the preceding claims, wherein the aripiprazole has a particle size distribution d(0.9) of about 300 µm or less.

7. The method according to any one of the preceding claims, wherein the diluent is calcium carbonate, calcium phosphate (dibasic and/or tribasic); calcium sulfate, powdered cellulose, dextrates, dextrin, fructose, kaolin, lactitol, anhydrous lactose, lactose monohydrate, maltose, mannitol, microcrystalline cellulose, sorbitol, sucrose, or starch.

8. The method according to claim 7, wherein the diluent is lactose monohydrate, microcrystalline cellulose, or starch.

9. The method according to any one of the preceding claims, wherein the diluent is present in an amount of about 35 to about 85 percent by weight of the tablet.

10. The method according to any one of the preceding claims, wherein the binders is acacia, alginic acid, carbomer, sodium carboxymethylcellulose, dextrin, ethylcellulose, gelatin, glucose, guar gum, hydroxypropyl cellulose, maltose, methylcellulose, polyethylene oxide, or povidone.

11. The method according to claim 10, wherein the binder is hydroxypropyl cellulose.

12. The method according to any one of the preceding claims, wherein the binder is present in an amount of about 0.5 to about 5 percent by weight of the tablet.

13. The method according to any one of the preceding claims, wherein the disintegrant is alginic acid, sodium croscarmellose, crospovidone, maltose, microcrystalline cellulose, potassium polacrilin, sodium starch glycolate, or starch.

14. The method according to claim 13, wherein the disintegrant is crospovidone, sodium starch glycolate or sodium croscarmellose.

15. The method according to any one of the preceding claims, wherein the disintegrant is present in an amount of about 3 to about 15 percent by weight of the tablet.

16. The method according to any one of the preceding claims, wherein the lubricant is calcium stearate, glyceryl behenate, magnesium stearate, mineral oil, polyethylene glycol, sodium stearyl fumarate, stearic acid, talc, or zinc stearate.

17. The method according to claim 16, wherein the lubricant is magnesium stearate.

18. The method according to any one of the preceding claims, wherein the lubricant is present in an amount of about 0.5 to about 2 percent by weight of the tablet.

19. The method according to one of the preceding claims, wherein the tablet has a dissolution rate where not less than 85% of the aripiprazole is dissolved after about 30 minutes.

20. The method according to claim 19, wherein the tablet has a dissolution rate where not less than 85% of the initial aripiprazole is dissolved after about 30 minutes.

21. The method according to claim 20, wherein the tablet has a dissolution rate where not less than 90% of the initial aripiprazole is dissolved after about 30 minutes.

22. A method of making an aripiprazole formulations according to claim 1 comprising:
blending aripiprazole Typo-I, lactose monohydrate, starch, color red, hydroxypropyl cellulose, and magnesium stearate into a mixture;
dry granulating the mixture and compressing the granulated mixture into slugs;
milling the slugs and blending the milled slugs with microcrystalline cellulose and magnesium stearate into a second mixture; and
compressing the second mixture into tablets,
wherein the tablets have a hardness range of about 9 to 15 Strong-Cobb units and a friability of less than about 1%.

23. The method according to claim 22, wherein the aripiprazole Type-I has a d(0.9) value of about 186 µm.

24. A dry formulation tablet made by a method according to any preceding claim.

## Patentansprüche

1. Verfahren zum Herstellen einer Formulierung wenigstens eines von wasserfreiem Aripiprazol Typ-I, Typ-II oder Form II, wobei das Verfahren folgendes umfaßt:
Bereitstellen eines Gemischs von Aripiprazol, wenigstens einem Verdünnungsmittel, wenigstens einem Tablettenbindemittel und wenigstens einem Tablettensprengmittel,
Vermischen des Gemischs unter Erhalt eines homogenen Gemischs,
optional Zugeben wenigstens eines Tablettengleitmittels zu dem homogenen Gemisch und
Trockenpressen des homogenen Gemischs zu der Formulierung.

2. Verfahren nach Anspruch 1, wobei die Formulierung eine Tablette ist.

3. Verfahren nach Anspruch 1, wobei die Formulierung ein Rohling oder ein Preßling ist.

4. Verfahren nach Anspruch 3, welches weiterhin das Mahlen des Rohlings oder Preßlings zu einem Granulat, das Zugeben wenigstens eines Tablettengleitmittels zu dem Granulat und das Trockenpressen des Granulats zu einer Tablette umfaßt.

5. Verfahren nach einem der vorangegangenen Ansprüche, wobei das Gemisch weiterhin einen Farbstoff umfaßt.

6. Verfahren nach einem der vorangegangenen Ansprüche, wobei das Aripiprazol eine Teilchengrößenverteilung d(0,9) von etwa 300 µm oder weniger hat.

7. Verfahren nach einem der vorangegangenen Ansprüche, wobei das Verdünnungsmittel Calciumcarbonat, Calciumphosphat (zweibasig und/oder dreibasig), Calciumsulfat, pulverförmige Zellulose, Dextrate, Dextrin, Fructose, Kaolin, Lactitol, wasserfreie Lactose, Lactosemonohydrat, Maltose, Mannitol, mikrokristalline Zellulose, Sorbitol, Saccharose oder Stärke ist.

8. Verfahren nach Anspruch 7, wobei das Verdünnungsmittel Lactosemonohydrat, mikrokristalline Zellulose oder Stärke ist.

9. Verfahren nach einem der vorangegangenen Ansprüche, wobei das Verdünnungsmittel in einer Menge von etwa 35 bis etwa 85 Prozent, bezogen auf das Gewicht der Tablette, vorliegt.

10. Verfahren nach einem der vorangegangenen Ansprüche, wobei das Bindemittel Akaziengummi, Alginsäure, Carbomer, Natriumcarboxymethylzellulose, Dextrin, Ethylzellulose, Gelatine, Glucose, Guargummi, Hydroxypropylzellulose, Maltose, Methylzellulose, Polyethylenoxid oder Povidon ist.

11. Verfahren nach Anspruch 10, wobei das Bindemittel Hydroxypropylzellulose ist.

12. Verfahren nach einem der vorangegangenen Ansprüche, wobei das Bindemittel in einer Menge von etwa 0,5 bis etwa 5 Prozent, bezogen auf das Gewicht der Tablette, vorliegt.

13. Verfahren nach einem der vorangegangenen Ansprüche, wobei das Sprengmittel Alginsäure, Natriumcroscarmellose, Crospovidon, Maltose, mikrokristalline Zellulose, Kaliumpolacrilin, Natriumstärkeglycolat oder Stärke ist.

14. Verfahren nach Anspruch 13, wobei das Sprengmittel Crospovidon, Natriumstärkeglycolat oder Natriumcroscarmellose ist.

15. Verfahren nach einem der vorangegangenen Ansprüche, wobei das Sprengmittel in einer Menge von etwa 3 bis etwa 15 Prozent, bezogen auf das Gewicht der Tablette, vorliegt.

16. Verfahren nach einem der vorangegangenen Ansprüche, wobei das Gleitmittel Calciumstearat, Glycerylbehenat, Magnesiumstearat, Mineralöl, Polyethylenglycol, Natriumstearylfumarat, Stearinsäure oder Zinkstearat ist.

17. Verfahren nach Anspruch 16, wobei das Gleitmittel Magnesiumstearat ist.

18. Verfahren nach einem der vorangegangenen Ansprüche, wobei das Gleitmittel in einer Menge von etwa 0,5 bis etwa 2 Prozent, bezogen auf das Gewicht der Tablette, vorliegt.

19. Verfahren nach einem der vorangegangenen Ansprüche, wobei die Tablette eine Auflösungsgeschwindigkeit hat, bei der nach etwa 30 Minuten nicht weniger als 85% des Aripiprazols gelöst sind.

20. Verfahren nach Anspruch 19, wobei die Tablette eine Auflösungsgeschwindigkeit hat, bei der nach etwa 30 Minuten nicht weniger als etwa 85% des initialen Aripiprazols gelöst sind.

21. Verfahren nach Anspruch 20, wobei die Tablette eine Auflösungsgeschwindigkeit hat, bei der nach etwa 30 Minuten nicht weniger als 90% des initialen Aripiprazols gelöst sind.

22. Verfahren zum Herstellen einer Aripiprazolformulierung nach Anspruch 1, welches folgendes umfaßt:
Vermischen von Aripiprazol Typ-I, Lactosemonohydrat, Stärke, rotem Farbstoff, Hydroxypropylzellulose und Magnesiumstearat zu einem Gemisch.
Trockengranulieren des Gemischs und Pressen des granulierten Gemischs zu Rohlingen,
Mahlen der Rohlinge und Vermischen der gemahlenen Rohlinge mit mikrokristalliner Zellulose und Magnesiumstearat zu einem zweiten Gemisch und
Pressen des zweiten Gemischs zu Tabletten,
wobei die Tabletten einen Härtebereich von etwa 9 bis 15 Strong-Cobb-Einheiten und eine Brüchigkeit von weniger als etwa 1 % haben.

23. Verfahren nach Anspruch 22, wobei das Aripiprazol Typ-I einen d(0,9)-Wert von etwa 186 µm hat.

24. Trocken formulierte Tablette, hergestellt durch ein Verfahren nach einem der vorangegangenen Ansprüche.

## Revendications

1. Procédé de réalisation d'une formulation d'au moins un composant parmi l'aripiprazole anhydre de type I, l'aripiprazole anhydre de type II ou l'aripiprazole anhydre de forme II, comprenant les étapes consistant à :
fournir un mélange d'aripiprazole, d'au moins un diluant, d'au moins un liant pour comprimé et d'au moins un délitant pour comprimé ;
brasser le mélange afin d'obtenir un mélange homogène ;
ajouter, en option, au moins un lubrifiant pour comprimé au mélange homogène ; et
comprimer à sec le mélange homogène dans la formulation.

2. Procédé selon la revendication 1, dans lequel la formulation est un comprimé.

3. Procédé selon la revendication 1, dans lequel la formulation est une pastille ou une substance compacte.

4. Procédé selon la revendication 3, comprenant en outre le broyage de la pastille ou de la substance compacte en un granulé, l'ajout d'au moins un lubrifiant pour comprimé au granulé, et la compression à sec du granulé dans un comprimé.

5. Procédé selon l'une quelconque des revendications précédentes, dans lequel le mélange comprend en outre un colorant.

6. Procédé selon l'une quelconque des revendications précédentes, dans lequel l'aripiprazole présente une granulométrie d(0,9) d'environ 300 µm ou moins.

7. Procédé selon l'une quelconque des revendications précédentes, dans lequel le diluant est du carbonate de calcium, du phosphate de calcium (dibasique et/ou tribasique), du sulfate de calcium, de la poudre de cellulose, des dextrates, de la dextrine, du fructose, du kaolin, du lactilol, du lactose anhydre, du monohydrate de lactose, du maltose, du mannitol, de la cellulose microcristalline, du sorbitol, du saccharose ou de l'amidon.

8. Procédé selon la revendication 7, dans lequel le diluant est du monohydrate de lactose, de la cellulose monocristalline ou de l'amidon.

9. Procédé selon l'une quelconque des revendications précédentes, dans lequel le diluant est présent en une quantité d'environ 35 à environ 85 pour cent en poids du comprimé.

10. Procédé selon l'une quelconque des revendications précédentes, dans lequel le liant est de l'acacia, de l'acide alginique, un carbomère, de la carboxyméthylcellulose de sodium, de la dextrine, de l'éthylcellulose, de la gélatine, du glucose, de la gomme de guar, de la cellulose hydroxypropylique, du maltose, de la méthylcellulose, de l'oxyde de polyéthylène ou de la povidone.

11. Procédé selon la revendication 10, dans lequel le liant est de la cellulose hydroxypropylique.

12. Procédé selon l'une quelconque des revendications précédentes, dans lequel le liant est présent en une quantité d'environ 0,5 à environ 5 pour cent en poids du comprimé.

13. Procédé selon l'une quelconque des revendications précédentes, dans lequel le délitant est de l'acide alginique, de la croscarmellose de sodium, du crospovidone, du maltose, de la cellulose microcristalline, de la polacriline de potassium, du glycolate d'amidon de sodium, ou de l'amidon.

14. Procédé selon la revendication 13, dans lequel le délitant est du crospovidone, du glycolate d'amidon de sodium ou de la croscarmellose de sodium.

15. Procédé selon l'une quelconque des revendications précédentes, dans lequel le délitant est présent en une quantité d'environ 3 à environ 15 pour cent en poids du comprimé.

16. Procédé selon l'une quelconque des revendications précédentes, dans lequel le lubrifiant est du stéarate de calcium, du béhénate de glycérol, du stéarate de magnésium, de l'huile minérale, du polyéthylène glycol, du fumarate de stéaryle sodique, de l'acide stéarique, du talc, ou du stéarate de zinc.

17. Procédé selon la revendication 16, dans lequel le lubrifiant est du stéarate de magnésium.

18. Procédé selon l'une quelconque des revendications précédentes, dans lequel le lubrifiant est présent en une quantité d'environ 0,5 à environ 2 pour cent en poids du comprimé.

19. Procédé selon l'une quelconque des revendications précédentes, dans lequel le comprimé présente un taux de dissolution auquel pas moins de 85 % de l'aripiprazole est dissous après environ 30 minutes.

20. Procédé selon la revendication 19, dans lequel le comprimé présente un taux de dissolution auquel pas moins de 85 % de l'aripiprazole initial est dissous après environ 30 minutes.

21. Procédé selon la revendication 20, dans lequel le comprimé présente un taux de dissolution auquel pas moins de 90 % de l'aripiprazole initial est dissous après environ 30 minutes.

22. Procédé de réalisation d'une formulation d'aripiprazole selon la revendication 1, comprenant les étapes consistant à :
brasser de l'aripiprazole de type I, du monohydrate de lactose, de l'amidon, un colorant rouge, de la cellulose hydroxypropylique et du stéarate de magnésium en un mélange ;
granuler à sec le mélange et comprimer le mélange granulé en pastilles ;
broyer les pastilles et brasser les pastilles broyées avec de la cellulose microcristalline et du stéarate de magnésium en un second mélange ; et
comprimer le second mélange en comprimés,
dans lequel les comprimés présentent une plage de dureté d'environ 9 à 15 unités de Strong-Cobb et une friabilité inférieure à environ 1 %.

23. Procédé selon la revendication 22, dans lequel l'aripiprazole de type I a une valeur d(0,9) d'environ 186 µm.

24. Comprimé à formulation sèche réalisée par un procédé selon l'une quelconque des revendications.
